# EUROPÄISCHE PATENTANMELDUNG

(11) **EP 3 417 766 A1**
(43) Veröffentlichungstag der Anmeldung: **26.12.2018**
(21) Anmeldenummer: 17001029.2
(22) Anmeldetag: 20.06.2017
(51) Int. Cl.: A61B 5/00, A47K 13/24, A61B 5/053, G01G 19/44, G01G 19/52

(54) **KLOBRILLE ZUR ERMITTLUNG VON KÖRPERWERTEN**

(71) Anmelder: Herbst, Martin, 5090 Lofer (AT)
(72) Erfinder: Herbst, Martin, 5090 Lofer (AT)

(57) **Zusammenfassung**

Die Erfindung ist eine Klobrille, in der Sensoren eingebaut sind, mit denen Messwerte des Körpers erfasst werden können.

## Beschreibung

Die Erfindung besteht aus einer Klobrille, in der Sensoren angebracht sind, mit denen Messwerte des Körpers erfasst werden können.
Wenn der Nutzer auf der Klobrille Platz nimmt, erfassen in der Klobrille eingebaute Sensoren Daten von der Körperoberfläche des Nutzers.
Diese Daten werden automationsunterstützt verarbeitet und in der Folge mitttels einer Anzeige auf der Erfindung dargestellt.
Die Messsensoren können alle technisch möglichen Werte erfassen, unter anderem Gewicht, Fettgehalt und Wassergehalt des Gewebse.
Diese Messwerte können an ein anderes Gerät (PC bzw. Rechner, sonstiges mobiles Gerät) gesendet und dort ausgewertet werden. Die ermittelten Daten geben dann Rückschlüsse über den augenblicklichen körperlichen Zustand desjenigen, der die Erfindung benützt hat hat. Der PC bzw. Rechner ermittelt ausgehend von eingegebenen Daten wie zB dem Alter, der Körpergröße eine Darstellung dieser Daten. Die verbundenen Geräte bieten auch die laufende Speicherung der ermittelten Daten und einen Vergleich über die Zeit an.

Die einzelnen Bestandteile der Erfindung sind, einzeln betrachtet, allgemein bekannt. Die technische Neuheit ist die Kombination dieser Teile in einer Klobrille, sodass der Anwender nicht mehrere unabhängige Geräte mit verschiedenen Sensoren verwenden muss und die Datenerfassung ganz einfach bei jeder Benutzung der Toilette erfolgen kann.

Mögliche Anwendungen ergeben sich im Bereich Wellness und Gesundheit. Aufgrund der ermittelten Daten und der automationsunterstützten Speicherung und Auswertung lässt sich der körperliche Zustand und die Fitness überwachen.

## Patentansprüche

1. Eine Klobrille, in der Sensoren angebracht sind, um Messwerte des Körpers (zB Gewicht, Fettgehalt, Wassergehalt und Eiweißgehalt des Gewebes) zu ermitteln.

2. Ein Gerät nach Anspruch 1, wobei für die automationsunterstützte Speicherung und Verarbeitung der Daten ein vom Gerät unabhängiger Rechner oder ein mobiles Endgerät verwendet wird, das mit dem Gerät über ein Kabel verbunden ist.

3. Ein Gerät nach Anspruch 1, wobei für die automationsunterstützte Datenverarbeitung ein vom Gerät unabhängiger Rechner oder ein mobiles Endgerät verwendet wird und die Übertragung der Daten über eine drahtlose Verbindung erfolgt, beispielsweise über Bluetooth, Zigbee, NFC oder Wireless LAN nach den verschiedenen Standards, beispielsweise 802.11 a, 802.11 ac, 802.11ad, 802.11 ah, 802.11 ax, 802.11b, 802.11g, 802.11n.

4. Ein Gerät nach Anspruch 1, wobei ein Vergleich der Körperwerte mit allgemein empfohlenen Werten erfolgt und daraus einfache Empfehlungen abgeleitet werden.

5. Ein Analysegerät nach Anspruch 1, wobei die ermittelten Daten offline gespeichert oder über das Internet zu einem Server übertragen werden, auf dem sie dann für den Nutzer gespeichert, ausgewertet und graphisch dargestellt werden.
